# EUROPÄISCHE PATENTSCHRIFT

(11) **EP 0 817 836 B1**
(45) Veröffentlichungstag und Bekanntmachung des Hinweises auf die Patenterteilung: **07.04.2004**
(21) Anmeldenummer: 96901343.2
(22) Anmeldetag: 30.01.1996
(51) Int. Cl.: C12N 5/06, C12N 7/00, C12Q 1/00

(54) **ZELLINIE UND VERFAHREN ZUR VERMEHRUNG VON TOLLWUTVIREN UND DEREN QUANTITATIVEN NACHWEIS**
CELL LINE AND PROCESS FOR REPLICATING RABIES VIRUSES AND THEIR QUANTITATIVE DETECTION
LIGNEE CELLULAIRE ET PROCEDE DE MULTIPLICATION DE VIRUS DE LA RAGE ET LEUR DETECTION QUANTITATIVE

(30) Priorität: 31.03.1995 DE 19512057
(43) Veröffentlichungstag der Anmeldung: 14.01.1998
(73) Patentinhaber: Aventis Behring Gesellschaft mit beschränkter Haftung, 35002 Marburg (DE)
(72) Erfinder: BERNHARDT, Dieter, D-35091 Cölbe (DE); GRÖNER, Albrecht, D-64342 Seeheim (DE)
(86) Internationale Anmeldenummer: PCT/EP1996/000424
(87) Internationale Veröffentlichungsnummer: WO 1996/030499

(56) Entgegenhaltungen:
- US-A- 4 664 912
- JOURNAL OF VIROLOGICAL METHODS, Bd. 27, 1990, AMSTERDAM, Seiten 277-286, XP000569715 CONSALES C.A. ET AL: "Cytopathic effect induced by rabies virus in McCoy cells" in der Anmeldung erwähnt
- FIELDS B.N. ET AL: "VIROLOGY" 1990 , RAVEN PRESS , NEW YORK XP002002025 17498 siehe Seite 890 - Seite 890

## Beschreibung

Die Erfindung betrifft eine permanente Zellinie und Verfahren zur Vermehrung von infektiösem Tollwutvirus und zu dessen quantitativem Nachweis mittels cytopathogenem Effekt (CPE) unter Verwendung dieser Zellinie sowie Verfahren zum Nachweis von Inhibitoren der Tollwutvirusvermehrung.

Zur Produktion von Poteinen und Virusantigenen werden lebende Zellen benötigt. Im Stand der Technik werden hierfür diploide Zellstämme oder permanente Zellinien verwendet, sofern sie für die Produktion der gewünschten Proteine oder Virusantigene geeignet sind. Permanente Zellinien haben gegenüber diploiden Zellstämmen den Vorteil, dass sie ein unbegrenztes Wachstum besitzen, d. h. sie sind immortalisiert. In weiten Passagebereichen weisen solche permanenten Zellinien gleichbleibende Eigenschaften hinsichtlich der Zell- und Antigenvermehrung auf, da bei ihnen keine Zelldifferenzierung wie bei diploiden Zellstämmen stattfindet. Neben der begrenzten Lebensspanne (Passagezahl) diploider Zellstämme ist ein weiterer schwerwiegender Nachteil, dass die Organe, Gewebe, sowie Hühnerembryonen, die als Ausgang für diploide Zellstämme benötigt werden, nicht in ausreichender Menge und zu jeder Zeit zur Verfügung stehen. Weiterhin kann das Ausgangsmaterial latent und/oder inapparent kontaminiert (Viren, Mycoplasmen, Bakterien) sein, wodurch eine optimal reproduzierbare Antigenproduktion nicht gewährleistet ist.

Bezüglich des Tollwutvirus ist zwar bekannt, dass sich dieses in Zellkulturen einer Vielzahl von Spezies vermehren lässt, aber in der Literatur existieren nur einige wenige Hinweise, dass dabei ein cytopathogener Effekt auftritt (Egert et al., Acta Virol. 33 (1989), 353 - 358, Consales et al., J. Virol. Meth. 27 (1990), 277 - 286, Campbell and Charlton, in: Development in veterinary virology: Rabies Kluwer Academic Publishers, Boston, Dordrecht, London, 1988). Bei dem cytopathogenen Effekt (CPE) handelt es sich um eine virusbedingte, spezifische Zellzerstörung (Lyse), die lichtmikroskopisch leicht nachweisbar ist.

Im allgemeinen verläuft die Tollwutvirusvermehrung in Gewebekulturen jedoch ohne CPE (Campbell und Charlton (1988)) bzw. virusbedingte cytopathogene Veränderungen sind nur unregelmäßig (Kaplan und Koprowski, Laboratory techniques in rabies, III. Edit. Wld. Hlth. Org. Monograph Serie 23, Geneva (1973)) oder treten lediglich transient auf (Smith et al., Intervirol. 8 (1977), 92 - 99), so dass ein verlässlicher Nachweis von Tollwutviren über Auswertung des CPE mit diesen Zellinien nicht möglich ist.

Der vorliegenden Erfindung lag somit das technische Problem zugrunde, eine permanente Zellinie bzw. Verfahren bereitzustellen, die diese Nachteile nicht aufweisen, d. h. die allgemein die Vermehrung infektiöser Tollwutviren mit verbesserter Ausbeute und unter Auftreten des cytopathogenen Effekts (CPE) erlauben, was deren sensitiven quantitativen Nachweis ermöglicht.

Die Lösung dieses technischen Problems erfolgt durch die Bereitstellung der in den Ansprüchen gekennzeichneten Ausführungsformen.

Es wurde überraschenderweise gefunden, dass in der erfindungsgemäßen permanenten Zellinie PH-2 Tollwutviren mit cytopathogenem Effekt (CPE) mit hoher Ausbeute vermehren. Andere Virusarten aus verschiedenen Virusfamilien wie Picornaviren, Herpesviren, Paramyxoviren, Reoviren und Togaviren lassen sich in dieser Zellinie ebenfalls vermehren. Diese Zellinie ist sowohl für die Produktion von Virusantigenen als auch für den Virus- und Antikörpernachweis geeignet. Besonders überraschend ist, dass sich in dieser Zellinie das zu den Rhabdoviren gehörende Tollwutvirus immer mit cytopathogenem Effekt (CPE) vermehren lässt, wodurch diese Zellinie sich besonders zum einfachen Nachweis und zur quantitativen Bestimmung von Tollwutvirus und (neutralisierenden) Tollwutvirusantikörpern eignet.

Somit betrifft die Erfindung die permanente Zellinie PH-2 und davon abgeleitete Zellinien, die Tollwutviren mit cytopathogenem Effekt vermehren. Die Zellinie PH-2 ist bei der DSM Deutsche Sammlung für Mikroorganismen in Braunschweig unter der Nummer DSM ACC 2165 hinterlegt.

In einer bevorzugten Ausführungsform betrifft die vorliegende Erfindung ein Verfahren zur Vermehrung von Tollwutviren mit cytopathogenem Effekt, das dadurch gekennzeichnet ist, daß die erfindungsgemäße Zellinie mit dem zu vermehrenden Virus infiziert und unter geeigneten Bedingungen bebrütet wird und nach Erreichen eines ausreichend hohen Titers an Viruspartikeln diese gewonnen und gereinigt werden.

Aus den so gewonnenen Viruspartikeln können nach im Stand der Technik bekannten Verfahren, z.B. durch Ultrazentrifugation, Ultrafiltration, Chromatographie u.ä. aus chemisch oder physikalisch desintegrierten Viruspartikeln Virusantigene gewonnen und gereinigt werden. Diese so erhaltenen Antigene können unter anderem zur Herstellung eines Impfstoffes oder für diagnostische Zwecke verwendet werden. In einer weiteren bevorzugten Ausführungsform betrifft die vorliegende Erfindung somit ein Verfahren zur Gewinnung von Tollwutviruspartikeln, das dadurch gekennzeichnet ist, daß nach Gewinnung der Viruspartikel aus diesen Virusantigene gewonnen und gereinigt werden.

Die Tatsache, daß mittels der erfindungsgemäßen Zellinie Tollwutviren nicht nur effizienter als mit im Stand der Technik bekannten Zellinien vermehren lassen, sondern auch bei gleichzeitig auftretendem cytopathogenem Effekt, erlaubt den sensitiven und einfachen Nachweis von Tollwutviren in Proben und deren quantitative Bestimmung.

Eine weitere bevorzugte Ausführungsform betrifft somit ein Verfahren zum Nachweis von Tollwutviren anhand des cytopathogenen Effekts, das dadurch gekennzeichnet ist, daß die erfindungsgemäße Zellinie mit einer das nachzuweisende Tollwutvirus vermutlich enthaltenden Probe in Kontakt gebracht wird, die Zellen bebrütet werden und das sich vermehrende Virus durch mikroskopische Ermittlung des CPE nachgewiesen wird. Zur Durchführung dieses Verfahrens wird beispielsweise die Virusprobe zu einer in Mikrotiterplatten oder anderen dem Fachmann bekannten Zellkulturgefäßen ausgesäten Zellkultur gegeben und entweder bis zum Ende des Versuches belassen oder nach einer vorgegebenen Adsorptionszeit mit dem Zellkulturmedium entfernt und durch frisches Zellkulturmedium ersetzt. Die Zellkulturen werden regelmäßig, beispielsweise bis zum 5. bis 7. Tag, nach der Inokulation lichtmikroskopisch hinsichtlich CPE untersucht.

In einer besonders bevorzugten Ausführungsform ist das erfindungsgemäße Nachweisverfahren dadurch gekennzeichnet, daß die Nachweisempfindlichkeit etwa eine log₁₀-Stufe höher liegt im Vergleich zum Stand der Technik (Fluoreszenz-Antikörpertechnik (FITC)).

Mittels der erfindungsgemäßen Zellinie können darüber hinaus auch Inhibitoren der Virusvermehrung bestimmt werden. Diese Inhibitoren können auf allen Stufen der Virusreplikation - Adsorption/Penetration, Virusnukleinsäure-Uncoating, Transkription und Translation sowie posttranslationaler Proteinmodifikation, Virus-Assembly und Ausschleusen (Budding) wirksam werden. Dabei kann es sich beispielsweise um neutralisierende Antikörper, chemische oder biologische Liganden gegen Virus- und Zellrezeptoren, Chemotherapeutika, Interferon oder andere Cytokine handeln. Die Inhibition der Virusvermehrung läßt sich durch das Ausbleiben eines CPE in der beanspruchten Zellinie einfach nachweisen. Die Versuchsdurchführung zum Nachweis einer Inhibition ist dem Fachmann bekannt.

Somit betrifft die vorliegende Erfindung auch ein Verfahren zum Nachweis von Inhibitoren der Tollwutvirusvermehrung, das dadurch gekennzeichnet ist, daß die Inhibition der Virusvermehrung in der erfindungsgemäßen Zellinie in Gegenwart des Inhibitors durch Ausbleiben des CPE nachgewiesen wird.

Die folgenden Beispiele erläutern die Erfindung.

### Beispiel 1

### Gewinnung der Zellinie PH-2

Zur Entwicklung der permanenten PH-2-Zellinie wurden primäre Pferdehaut-Zellen mit wenigen, gleichzeitig im Labor vermehrten permanenten Affennierenzellen (Vero-M) gemischt. Dabei ergab sich überraschenderweise, daß morphologisch von den Vero-Zellen unterscheidbare Zellen die Pferdehaut-Fibroblasten überwuchsen. Diese Zellen wurden isoliert und als PH-2-Zelle bezeichnet.

Die Karyotypisierung der PH-2-Zelle, im Vergleich zur Vero-M-Zelle, ist in Tabelle 1 dargestellt. Die PH-2-Zelle weist einen von der Vero-M-Zelle unterschiedlichen Chromosomensatz sowie ein unterschiedliches Chromosomenverteilungsmuster auf.

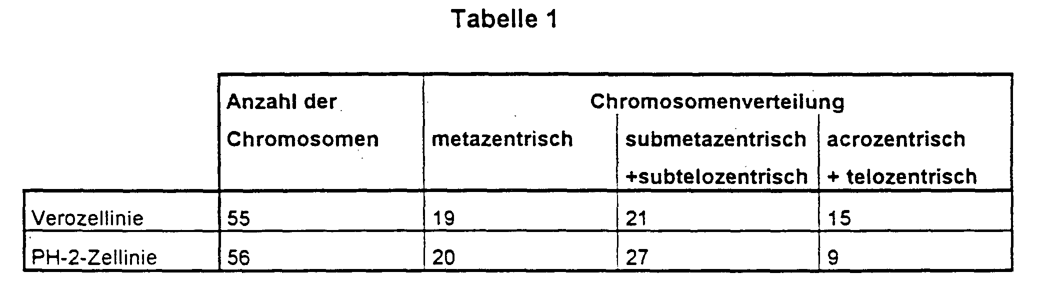

In der PH-2-Zellinie vermehrt sich das Tollwutvirus im Gegensatz zur Ausgangszellinie (Vero) mit CPE.

### Beispiel 2

### Vergleichstitration von Tollwutvirus, Stamm Flury LEP (ATCC VR-138), in Hühnerfibroblastenkulturen (Standardmethode) und PH-2-Zellkulturen.

Das in Hühnerfibroblastenkulturen vermehrte Virus wurde aus der Produktion der Behringwerke AG erhalten.

Hühnerfibroblastenzellen wurden entsprechend der Vorschrift 24 Std. vor Titrationsauftrag in Mikrotiterplatten ausgesät. Die Anlage der Mikrotiterplatten mit PH-2-Zellen erfolgte unmittelbar vor Titrationsbeginn. Als Medium für die Zellaussaat wurde bei den Hühnerfibroblasten Medium 3 mit 1 % fötalem Kälberserum (FKS) und NSP, für die PH-2-Zellen EME-Medium und 2% FKS und NSP verwendet.
Das Tollwutvirus wurde in log₁₀-Stufen in Medium 3 verdünnt. Ausgehend von jeweils einer Verdünnung wurden 8 Näpfe/Verdünnungsstufe der entsprechenden Kulturart mit 200µl/Napf beimpft. Anschließend erfolgte die Bebrütung der Kulturen im CO₂-Brutschrank bei 37°C.

Die Titerablesung erfolgte in den Hühnerfibroblastenkulturen nach Anfärbung der Zellen mit FITC markierten Antikörpern (Fluoreszenzmethode) 3 bis 5 Tage nach Testansatz, bei den PH-2-Kulturen mikroskopisch mittels des vom Tollwutvirus verursachten CPE am 5. bis 7. Tag nach Testbeginn. Die Titerberechnung erfolgte nach der Methode von Kärber.

Die Ergebnisse der Vergleichstitrationen sind nachfolgend in Tabelle 2 aufgeführt.

**Tabelle 2**

| **Versuchs-Nr.** | **Titer in log**_{**10**}**/ml** | |
|---|---|---|
| | **in Hühnerfibroblasten** | **in PH-2-Zellen** |
| 1 | 8.0 | 9.0 |
| 2 | 7.7 | 8.8 |
| 3 | 7.5 | 8.6 |
| 4 | 7.9 | 8.2 |
| 5 | 7.1 | 8.0 |
| 6 | 6.7 | 7.5 |
| 7 | 6.0 | 7.2 |
| 8 | 4.8 | 6.0 |
| 9 | 4.1 | 5.2 |
| 10 | 3.0 | 4.8 |
| 11 | 2.3 | 4.0 |
| Testauswertung | Fluoreszenz-Antikörpertechnik | CPE |

Wie aus den Vergleichsuntersuchungen hervorgeht, sind die in PH-2-Zellen durch mikroskopischen Nachweis ermittelten Titer im Schnitt um eine log₁₀-Stufe höher als die in Hühnerfibroblasten gefundenen Titer. Damit zeigt sich, daß neben der erheblichen Verfahrensvereinfachung des Tollwutvirusnachweises diese Methode auch wesentlich sensitiver gegenüber den im Stand der Technik verfügbaren Nachweisverfahren ist.

### Beispiel 3

### Tollwutvirusvermehrung in PH-2-Zellen (Vermehrungskurve)

PH-2-Zellen dicht gewachsener Rouxschalen wurden nach Mediumwechsel in EME-Medium ohne Serum mit Tollwutvirus (MOI (multiplicity of infection:0.01) infiziert und bei 37°C bebrütet. Nach den in der Tabelle 3 angegebenen Zeitabständen wurden aus der infizierten Rouxschale Proben entnommen und der Gehalt an infektiösen Viruspartikeln bestimmt. Die gefundenen Werte sind nachfolgend in Tabelle 3 aufgeführt.

**Tabelle 3**

| | **0** | **1** | **3** | **4** | **5** | **6** | **7** |
|---|---|---|---|---|---|---|---|
| **Titer/ml in log**_{**10**} | 4.5 | 4.5 | 7.4 | 7.5 | 8.0 | 8.3 | 7.4 |

Wie aus den Werten von Tabelle 3 hervorgeht, läßt sich Tollwutvirus mit hohen Titern in der PH-2-Zellinie vermehren. Somit ist diese Zellinie für die Tollwutvirusantigenproduktion sehr gut geeignet.

### Beispiel 4

### Prüfung der Titer-Reproduzierbarkeit beim Tollwutvirus in verschiedenen PH-2-Passagen

Ausgehend von der gleichen Tollwutviruspräparation, Stamm Flury LEP, wurde der Titer in der 36., 85. und 100. Passage der PH-2-Zellen bestimmt.

Die nachfolgende Tabelle 4 beinhaltet die Resultate der Versuchsreihen.

**Tabelle 4**

| **PH-2** | **Tollwutvirustiter in log**_{**10**}**/ml** | | |
|---|---|---|---|
| **Passage** | **Versuch 1** | **Versuch 2** | **Versuch 3** |
| 36 | 6.9 | 6.9 | 7.1 |
| 85 | 6.5 | 6.9 | 7.1 |
| 100 | n.u. | 6.9 | 6.9 |
| n.u. = nicht untersucht | | | |

Wie aus den Ergebnissen dieser Tabelle hervorgeht, zeigt die PH-2-Zellinie in den geprüften Passagebereichen, zwischen 36. und 100. Passage, gegenüber Tollwutvirus eine unveränderte Sensitivität.

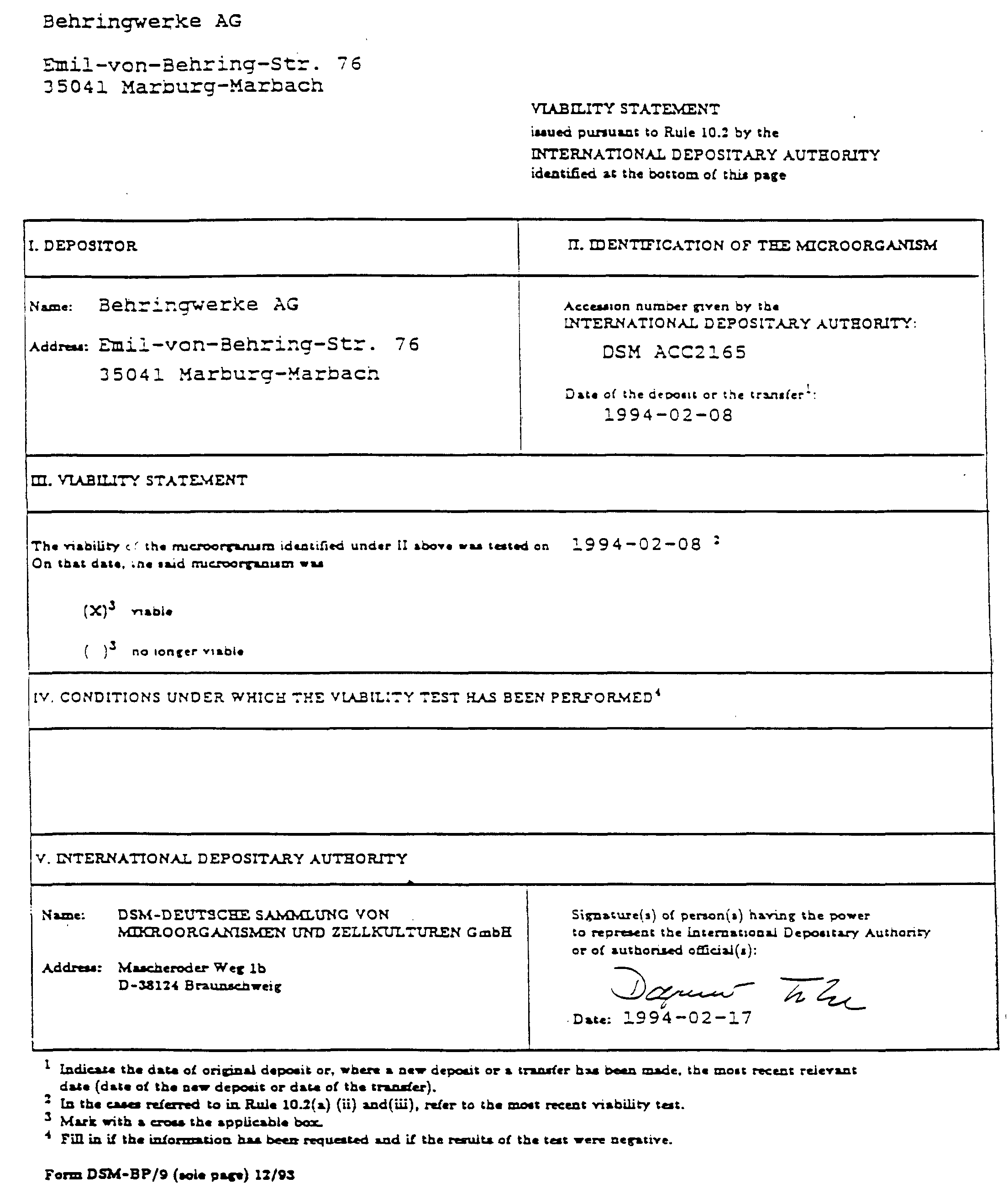
Behringwerke AG

Emil-von-Behring-Str. 76
35041 Marburg-Marbach

VIABILITY STATEMENT
issued pursuant to Rule 10.2 by the
INTERNATIONAL DEPOSITARY AUTHORITY
identified at the bottom of this page

I. DEPOSITOR | II. IDENTIFICATION OF THE MICROORGANISM

Name: Behringwerke AG | Accession number given by the
Address: Emil-von-Behring-Str. 76 | INTERNATIONAL DEPOSITARY AUTHORITY:
35041 Marburg-Marbach | DSM ACC2165
| Date of the deposit or the transfer1:
| 1994-02-08

III. VIABILITY STATEMENT

The viability of the microorganism identified under II above was tested on 1994-02-08 2
On that date, the said microorganism was

(X)3 viable

( )3 no longer viable

IV. CONDITIONS UNDER WHICH THE VIABILITY TEST HAS BEEN PERFORMED4

V. INTERNATIONAL DEPOSITARY AUTHORITY

Name: DSM-DEUTSCHE SAMMLUNG VON | Signature(s) of person(s) having the power
MIKROORGANISMEN UND ZELLKULTUREN GmbH | to represent the International Depositary Authority
Address: Mascheroder Weg 1b | or of authorised official(s):
D-38124 Braunschweig |
| Date: 1994-02-17

1 Indicate the date of original deposit or, where a new deposit or a transfer has been made, the most recent relevant
date (date of the new deposit or date of the transfer).
2 In the cases referred to in Rule 10.2(a) (ii) and(iii), refer to the most recent viability test.
3 Mark with a cross the applicable box.
4 Fill in if the information has been requested and if the results of the test were negative.

Form DSM-BP/9 (sole page) 12/93

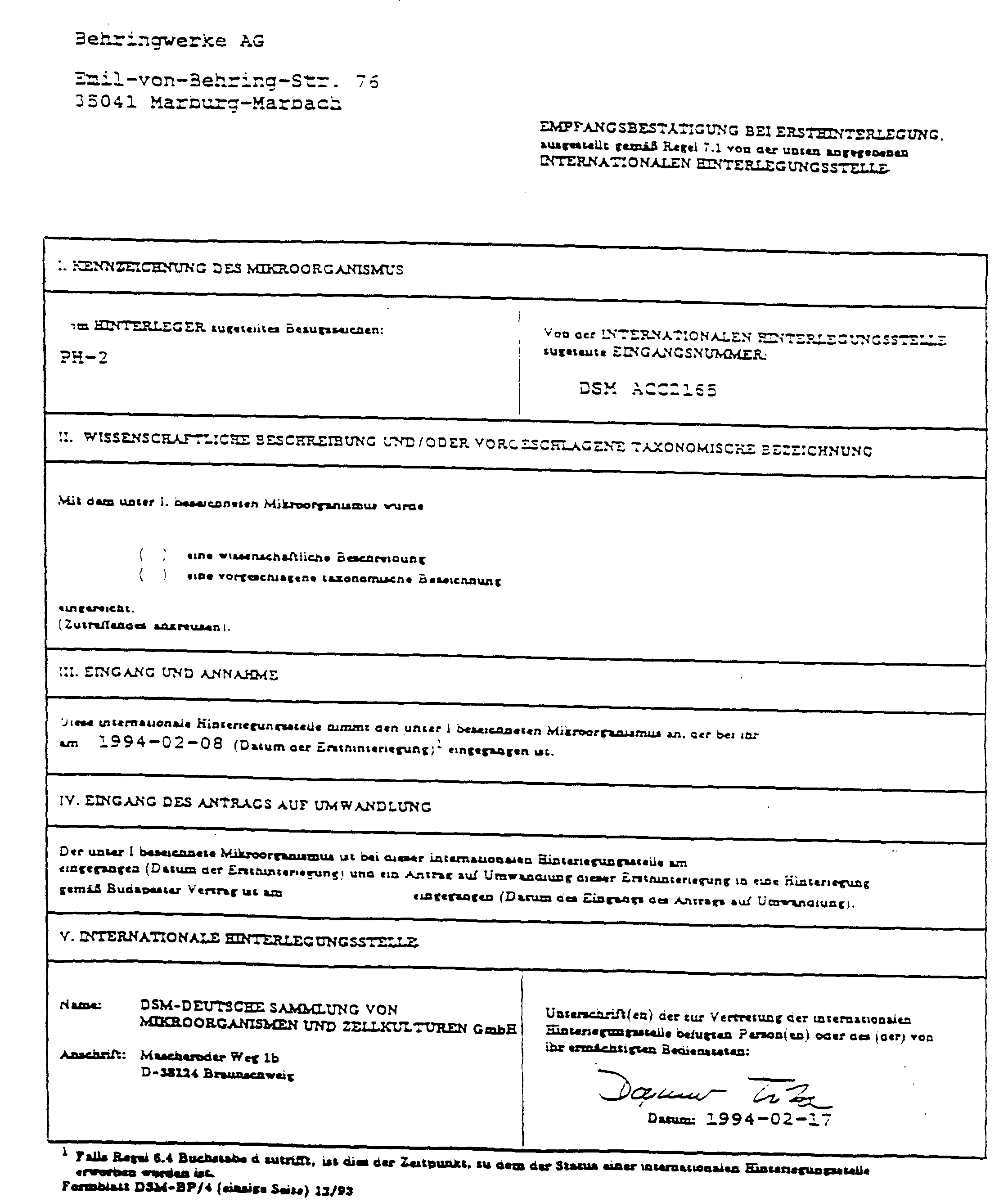
Behringwerke AG

Emil-von-Behring-Str. 76
35041 Marburg-Marbach

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen: | Von der INTERNATIONALEN HINTERLEGUNGSSTELLE zugeteilte EINGANGSNUMMER:
PH-2 | DSM ACC2165

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr
am 1994-02-08 (Datum der Ersthinterlegung)¹ eingegangen ist.

IV. EINGANG DES ANTRAGS AUF UMWANDLUNG

Der unter I bezeichnete Mikroorganismus ist bei dieser internationalen Hinterlegungsstelle am
eingegangen (Datum der Ersthinterlegung) und ein Antrag auf Umwandlung dieser Ersthinterlegung in eine Hinterlegung
gemäß Budapester Vertrag ist am eingegangen (Datum des Eingangs des Antrags auf Umwandlung).

V. INTERNATIONALE HINTERLEGUNGSSTELLE

Name: DSM-DEUTSCHE SAMMLUNG VON MIKROORGANISMEN UND ZELLKULTUREN GmbH | Unterschrift(en) der zur Vertretung der internationalen Hinterlegungsstelle befugten Person(en) oder des (der) von ihr ermächtigten Bediensteten:
Anschrift: Mascheroder Weg 1b D-38124 Braunschweig |
 | Datum: 1994-02-17

¹ Falls Regel 6.4 Buchstabe d zutrifft, ist dies der Zeitpunkt, zu dem der Status einer internationalen Hinterlegungsstelle erworben worden ist.
Formblatt DSM-BP/4 (einzige Seite) 12/93

## Patentansprüche

1. Permanente Zellinie mit der Bezeichnung PH-2 (Hinterlegungs-Nr. DSM ACC 2165) oder davon abgeleitete Zellinien, die Tollwutviren mit cytopathögenem Effekt (CPE) vermehren.

2. Verfahren zur Vermehrung von Tollwutviren mit cytopathogenem Effekt, **dadurch gekennzeichnet, dass** die Zellinie nach Anspruch 1
a) mit dem zu vermehrenden Virus infiziert wird,
b) die Zellen bebrütet werden; und
c) nach Erreichen eines ausreichend hohen Titers an Viruspartikeln diese gewonnen und gereinigt werden.

3. Verfahren nach Anspruch 2, weiter **dadurch gekennzeichnet, dass** nach Schritt c) die Virusantigene aus den Viruspartikeln gewonnen und gereinigt werden.

4. Verfahren zum Nachweis von Tollwutviren anhand des cytopathogenen Effekts, verursacht durch die Virusvermehrung in der Zellinie nach Anspruch 1, **dadurch gekennzeichnet, dass**
a) die Zellinie mit einer das nachzuweisende Tollwutvirus vermutlich enthaltenden Probe in Kontakt gebracht wird;
b) die Zellen bebrütet werden; und
c) das Virus nach Beginn der Bebrütung durch mikroskopische Ermittlung des durch die Virusinfektion bedingte CPE bestimmt wird.

5. Verfahren nach Anspruch 4, weiter **dadurch gekennzeichnet, dass** die Nachweisempfindlichkeit etwa eine log₁₀-Stufe höher ist im Vergleich zur Fluoreszenz-Antikörpertechnik (FITC).

6. Verfahren zum Nachweis von Inhibitoren der Tollwutvirusvermehrung, **dadurch gekennzeichnet, dass** die Inhibition der Virusvermehrung in der Zellinie nach Anspruch 1 in Gegenwart des Inhibitors durch Ausbleiben des CPE nachgewiesen wird.

## Claims

1. A permanent cell line having the designation PH-2 (deposition no. DSM ACC2165), or a cell line derived therefrom which replicates rabies viruses which have a cytopathic effect (CPE).

2. A process for replicating rabies viruses which have a cytopathic effect, which comprises
a) infecting the cell line as claimed in claim 1 with the virus to be replicated;
b) incubating the cells; and
c) isolating and purifying virus particles after they have reached a sufficiently high titer.

3. The process as claimed in claim 2, wherein, furthermore, after step c), the viral antigens are isolated from the virus particles and purified.

4. A process for detecting rabies viruses on the basis of the cytopathic effect which is caused by replication of the virus in the cell line as claimed in claim 1, which comprises
a) bringing the cell line into contact with a sample which is suspected of containing the rabies virus to be detected;
b) incubating the cells; and
c) determining the virus, after commencing the incubation, by means of microscopically ascertaining the CPE which is caused by the virus infection.

5. The process as claimed in claim 4, wherein, furthermore, the sensitivity of detection is approximately one log₁₀ step higher as compared with the fluorescence-antibody technique (FITC).

6. A process for detecting inhibitors of rabies virus replication, which comprises detecting inhibition of the replication of the virus in the cell line as claimed in claim 1, in the presence of the inhibitor, by the absence of the CPE.

## Revendications

1. Lignée cellulaire permanente nommée PH-2 (N° de dépôt DSM ACC 2165) ou lignée cellulaire qui en est dérivée, qui multiplient le virus de la rage ayant un effet cytopathogène (CPE).

2. Procédé de multiplication du virus de la rage ayant un effet cytopathogène, **caractérisé en ce que** la lignée cellulaire selon la revendication 1
a) est infectée par le virus à multiplier,
b) les cellules sont incubées ; et
c) après obtention d'un titre suffisamment élevé de particules virales, celles-ci sont récoltées et purifiées.

3. Procédé selon la revendication 2, **caractérisé en outre en ce qu'**après l'étape c) les antigènes viraux sont extraits des particules virales et sont purifiés.

4. Procédé de détection du virus de la rage à l'aide de l'effet cytopathogène, causé par la multiplication du virus dans la lignée cellulaire selon la revendication 1, **caractérisé en ce que**
a) la lignée cellulaire est mise en contact avec un échantillon supposé contenir le virus de la rage devant être détecté ;
b) les cellules sont incubées ; et
c) le virus est dosé après le début de l'incubation par évaluation microscopique du CPE causé par l'infection virale.

5. Procédé selon la revendication 4, **caractérisé en outre en ce que** la sensibilité de détection est environ supérieure d'un degré log₁₀ à la sensibilité de la technique par fluorescence avec anticorps (FITC).

6. Procédé de détection d'inhibiteurs de la multiplication du virus de la rage, **caractérisé en ce que** l'inhibition de la multiplication du virus dans la lignée cellulaire selon la revendication 1 est détectée en présence de l'inhibiteur par l'arrêt du CPE.
